# EUROPÄISCHE PATENTSCHRIFT

(11) **EP 3 338 843 B1**
(45) Veröffentlichungstag und Bekanntmachung des Hinweises auf die Patenterteilung: **03.07.2024**
(21) Anmeldenummer: 17002035.8
(22) Anmeldetag: 15.12.2017
(51) Int. Cl.: A61M 16/00

(54) **VORRICHTUNG ZUR BEATMUNG MIT VORGABE EINES PATIENTEN-INDIVIDUELLEN DRUCKPROFILS**
VENTILATION DEVICE WITH SPECIFICATION OF A PATIENT-SPECIFIC PRESSURE PROFILE
DISPOSITIF RESPIRATOIRE À SPÉCIFICATION D'UN PROFIL DE PRESSION INDIVIDUEL DU PATIENT

(30) Priorität: 23.12.2016 DE 102016015441
(43) Veröffentlichungstag der Anmeldung: 27.06.2018
(73) Patentinhaber: Löwenstein Medical Technology S.A., 2557 Luxembourg (LU)
(72) Erfinder: Dömer, Benno, 73275 Ettlingen (DE); Alshut, Rüdiger, 76131 Karlsruhe (DE); Schwaibold, Matthias, 76228 Karlsruhe (DE)
(74) Vertreter: Marx, Thomas

(56) Entgegenhaltungen:
- EP-A1- 1 518 579
- EP-A2- 1 129 742
- EP-A2- 1 136 094
- DE-A1-102007 039 004
- DE-A1-102009 041 247
- US-A1- 2003 121 519
- US-A1- 2011 226 248
- US-A1- 2011 245 704
- US-A1- 2014 182 589

## Beschreibung

Bislang gibt es bei der Beatmung typischerweise ein (CPAP, APAP), zwei (Bilevel, ASV) oder drei (TRILevel) Druckniveaus, zwischen denen anhand bestimmter Atmungscharakteristiken an Umschaltpunkten hin und her geschaltet wird. Die Umschaltung kann dabei stufenförmig oder durch lineare oder nichtlineare Rampen erfolgen.

Diese Beatmungsformen sind recht starr und können nur mit wenigen Freiheitsgraden auf das individuelle Atemmuster des Patienten und die Therapie seiner individuellen respiratorischen Erkrankung eingehen.

### Stand der Technik

Aus dem Dokument EP 1 518 479 A1 ist ein Verfahren zu Steuerung eines Beatmungsgerätes bekannt, wobei während einer Analysephase messtechnisch wenigstens ein Ist-Parameter einer spontanen Patientatmung erfasst wird. Zudem ist eine Vorrichtung zur Beatmung zur Durchführung des Verfahrens bekannt.

Aus dem Dokument US 2011/226 218 A1 ist ein Verfahren zum Bereitstellen einer Druckunterstützung für einen Patienten bekannt, dass das Bestimmen eines Maßes einschließt, das mit einer Inspirationszeit des Patienten während der Therapie verbunden ist.

Aus dem Dokument De 10 2009 041 247 A1 ist ein Verfahren für eine kontrollierte Beatmung bekannt. Dabei wird eine Applikation des Atemgases durch die Beatmungseinrichtung auf zwei unterschiedlichen Atemwegsdruckniveaus mit einem oberen und einem unteren Niveau durchgeführt.

Aus dem Dokument EP 1 129 742 A2 ist ein Beatmungsgerät bekannt, dessen Servosteuerung den Unterstützungsgrad durch Einstellen des Profils der Druckwellenform sowie der Druckmodulationsamplitude einstellt.

Aus dem Dokument US 2003/121 519 A1 ist ein Beatmungsgerät bekannt, die eine Soll-Druck-Funktion auf Basis eines ermittelten Flow-Verlaufs auf Basis einer mathematischen Abbildung berechnet. Die Soll-Druck-Funktion hängt von Parametern ab, die auf Basis von vergangenen Flow- bzw. Volumen-Verläufen angepasst werden. Dieses Dokument offenbart die Detektion einer Hypopnea durch Vergleich von Kurzzeit- und Langzeit-Durchschnittswerten von maximalem Flowrate-Werten, auf deren Basis die Parameter der Soll-Druck-Funktion angepasst werden. Die Parameter der Soll-Druck-Fuktion können auch unter Berücksichtigung von Benutzereingaben zu bestimmen. Applikation einer Druckrampe wird auch in diesem Dokument beschrieben.

Die Aufgabe der Erfindung ist daher, eine individuelle Anpassung an den Patientenbedarf zu ermöglichen.

Die Aufgabe wird gelöst durch eine Vorrichtung nach Anspruch 1. Die Unteransprüche definieren erfindungsgemäße Ausführungsbeispiele.

Beschrieben wird eine Vorrichtung zur Beatmung mit zumindest einer Druckgasquelle für Atemgas und einer Sensoreinheit zur Bestimmung von Atemgas-Druck und/oder Fluss /-Volumen und einer Steuereinheit. Die Steuereinheit zeichnet ein individuelles Atemmuster eines Patienten auf und bildet daraus ein für den Patienten individuelles Druckprofil, welches von der Druckgasquelle vorgegeben wird.

Es ist möglich, die Atmung des Patienten mit hoher Abtastrate (mindestens 40 Hz, bevorzugt > 60 Hz), quasi kontinuierlich, zu messen und daraufhin ein laufend angepasstes Druckprofil zu applizieren, das aus einer Vielzahl von vorgegebenen Druckniveaus besteht. IPAP und EPAP sind dabei als Begriffe höchstens noch für Minimum und Maximum des Druckprofils anwendbar. Auch einen klassischen Trigger gibt es nicht mehr unbedingt, die Synchronität aus Druckprofil und Atmungsprofil kann laufend und iterativ optimiert werden. Das Gerät lernt also laufend, den Beginn des nächsten Atemzuges voraus zu sagen.

Die zugehörige Vorrichtung umfasst neben den üblichen Teilen (Druckquelle, mindestens ein Schlauch, Patienteninterface, Drucksteuerung, Energieversorgung, Schnittstellen u.a. für die Cloud, Sensoren für Signale, die mit Druck, Fluss oder Volumen in Zusammenhang stehen,...) beispielsweise folgende charakterisierenden Punkte der Drucksteuerung:
Optional, Alternativ oder Ergänzend können eine oder mehrere der folgenden Ausführungsmöglichkeiten umgesetzt sein.
1) Verlaufs-Analysator, der den Verlauf von mindestens einem Signal, das mit Druck, Fluss oder Volumen in Zusammenhang steht, kontinuierlich (mindestens mit 1 Hz, bevorzugt > 20 Hz, besonders bevorzugt > 50 Hz) abtastet, analysiert und zwischenspeichert.
2) Ausschneide-Einheit, die definierte oder aus dem Verlauf erkannte Zeiträume des Verlaufes mindestens eines Signals aus dem Zwischenspeicher an die Atemmuster-Analysator und/oder den Kontur-Analysator übergibt
3) Kontur-Analysator, der aus dem Verlauf des aktuellen Zeitraums, der ihm von der Ausschneide-Einheit aktiv oder passiv übergeben wurde, das Vorhandensein oder Nicht-Vorhandensein bestimmter akuter Atmungszustände oder Beatmungszustände erkennt
4) Atemmuster-Analysator, der das typische, individuelle Atemmuster eines Patienten aus mindestens 2 Verläufen ermittelt, die ihm von der Ausschneide-Einheit aktiv oder passiv übergeben wurden.
5) Transformations-Funktion, die durch mindestens eine vorgegebene mathematische Abbildung das individuelle Atemmuster in ein für den aktuellen oder darauffolgenden Atemzug gültiges Druckprofil überführt.
6) Druckprofil-Applikator, der anhand der Ergebnisse des Verlaufs-Analysators das vorgesehene Druckprofil auf den augenblicklichen Verlauf des Atemzuges optimiert und daraus den augenblicklichen Solldruck kontinuierlich (mindestens 1 Hz, bevorzugt > 20 Hz, besonders bevorzugt > 50 Hz) an die Druckquelle übergibt.

### Verlaufs-Analysator;

Eine beispielhafte nicht abschließende Liste von möglichen Signaltypen, die der Verlaufs-Analysator analysiert sind: Gerätefluss, Patientenfluss, Patientenfluss mit Spülfluss, Druck am Geräteausgang, Druck in der Maske, Druck in den Atemwegen, aktuelles Tidalvolumen, aktuelles alveoläres Tidalvolumen sowie Schätzgrößen für eines dieser Signale oder damit in Zusammenhang stehende Signale.

Eine beispielhafte nicht abschließende Liste von möglichen Analysen die der Verlaufs-Analysator durchführt sind: Erkennung der Atemphase, Erkennung des aktuellen Momentes innerhalb des Verlaufes eines Atemzuges, Erkennung von steigendem oder fallendem Volumen gegenüber dem individuellen Atemmuster oder den unmittelbar vorangegangenen Atemzügen, Synchronität von Druckverlauf und Patientenatmung.

### Ausschneide-Einheit

- Beginn und Ende der Zeiträume werden definiert durch beispielsweise: feste Zeitdauern, beispielsweise feste Sekundenanzahl oder 1 / Atemfrequenz, oder durch charakteristische Punkte wie Nulldurchgang mindestens eines der analysierten Signale nach Abzug eines Offsets, Inspirationsbeginn, Exspirationsbeginn, inspiratorische PeakFluss, exspiratorischer PeakFluss, Erreichen eines vorgegebenen Druckwertes, maximale oder minimale Steigung eines Signals oder Nulldurchgang der Steigung, maximale oder minimale Krümmung des Signals oder Nulldurchgang, Beginn der Ende eines kompletten Atemzuges, Erreichen eines bestimmten Volumens, ...

### Kontur-Analysator;

Eine beispielhafte nicht abschließende Liste von möglichen erkannten Atmungszuständen oder Beatmungszustände:
- Flattening in der Inspiration durch Obstruktion der oberen Atemwege
- Flattening oder Instabilitäten in der Exspiration durch Obstruktion der unteren Atemwege, Sekret, COPD
- Masken- oder Mundleckage
- Verpasste Atemzüge
- Artefaktzustände wie Bewegung, Sprechen, Husten, Schlucken, ...
- Synchronität bzw. aktueller Zeitversatz von Druckverlauf und Atmungsverlauf
- Aktuelle Atemanstrengung
- Aktuelles aktives Wehren gegen die Beatmung
- Aktuelles Tidalvolumen, alveoläres Tidalvolumen, Minutenvolumen, alveoläres Minutenvolumen (alveoläre Volumina durch Berücksichtigung eines ventilatorischen Totraums, der manuell vorgegeben oder vom Gerät automatisch geschätzt wird)
- Aktueller Volumenbedarf
- Resistance und/oder Compliance der Atemwege und Lunge oder damit in Zusammenhang stehende Werte

Aus mindestens 2 einzelnen Atemmusterverläufen wird das individuelle Atemmuster des Patienten bestimmt durch beispielsweise mindestens einen der folgenden Schritte
- Normierung von aktuellen Atemmustern in Zeit und Fluss oder Volumen
- Mittelung von einzelnen Atemmustern
- Gewichtete Mittelung von einzelnen Atemmustern, wobei die Gewichtung abhängt beispielsweise von Dauer, Peakfluss, mittlerem Fluss, Volumen oder dem den vom Kontur-Analysator erkannten Zuständen (beispielsweise niedriges Gewicht bei Auftreten von Flattening oder Artefakten, ...)
- Least-Squares Anpassung oder artverwandte Anpassung einer aktuellen Schätzung des individuellen Atemmusters an das tatsächliche Atemmuster der aktuellen Zeiteinheit
- Gleitender Mittelwert des Verlaufes mehrerer Zeiteinheiten
- Beseitigung eines Zeitversatzes von Atemmustern innerhalb von mindestens 2 Zeiteinheiten
- Glättung des Atemmusters, beispielsweise zur Beseitigung von Verfälschungen, beispielsweise durch Flattening

Die mathematische Abbildung kann beispielsweise beinhalten:
- Vorgaben aufgrund des Krankheitsbildes, beispielsweise optimiertes Druckprofil bei restriktiven oder obstruktiven Lungen; das Krankheitsbild kann dabei entweder vom Anwender vorgegeben, oder vom Beatmungsgerät automatisch erkannt werden
- Vorgaben zum Therapieziel, beispielsweise Minimierung der Atemarbeit, Stabilisierung des CO2-Wertes, Optimierung des Patientenkomforts, Vermeidung eines intrinsischen PEEPs bei der Beatmung, Einhaltung eines Zielwertes für Tidalvolumen, alveoläres Tidalvolumen, Minutenvolumen, alveoläres Minutenvolumen, Therapie von Obstruktionen der oberen Atemwege, Stabilisierung des SpO2-Wertes, Sicherstellung eines minimalen oder maximalen Druckhubes, Vermeidung von gefährdenden Situationen, ...; die Vorgaben können dabei vom Anwender gewählt oder fest im Gerät hinterlegt sein
- Fest im Gerät hinterlegte Universalfunktionen zur Überführung eines Atemmusters in ein passendes Druckprofil
- Maßnahmen zur Optimierung der Synchronität von Druckprofil und Atemanstrengung des Patienten, beispielsweisedurch Strecken oder Stauchen des Druckprofils
- Maßnahmen zur Beschleunigung der Atmung oder Verlangsamung der Atmung
- Dabei kann die Transformations-Funktion optional auf weitere Signale von außen zugreifen, insb.: SpO2, CO2, Pulswelle, Effort, Herzfrequenz, Eingaben durch Patient oder medizinisches Personal, EMG der Atemmuskeln, Atemgeräusche, Atemanstrengung
- Oder auf die Ergebnisse des Kontur-Analysators, um beispielsweisedurch Anhebung / Absenkung des Druckprofils auf Obstruktionen der oberen Atemwege zu reagieren, eines der genannten Volumina zu kontrollieren oder durch Beschleunigen / Verlangsamen des Druckprofils die Synchronität von Beatmung und Atemanstrengung des Patienten zu verbessern oder intrinsischen PEEP zu verringern

Der Druckprofil-Applikator optimiert das Druckprofil für den aktuellen Atemverlauf anhand der Ergebnisse des Verlaufs-Analysators:
- Insbesondere um auf Abweichungen des aktuellen Atmungsverlaufes vom individuellen Atemmuster einzugehen
- Die Optimierung kann beispielsweise derart erfolgen, dass die Abweichung unterstützt oder ihr entgegenwirkt wird
- Abweichungen werden insbesondere bezüglich Höhe von Fluss/Volumen, alveolärem Fluss/Volumen oder Beschleunigung / Verlangsamung der Atmung bestimmt
- Die Optimierung erfolgt beispielsweise durch eine mathematische Abbildung, insbesondere anhand der Ergebnisse des Kontur-Analysators
- Oder durch Skalierung des durch die mathematische Abbildung ermittelten Druckprofils in Höhe oder Dauer / Geschwindigkeit

Die Steuereinheit (16) ist so eingerichtet und ausgebildet, dass sie als Funktionseinheiten aufweist einen Verlaufs-Analysator (18) und (optional) eine Ausschneide-Einheit (19) und einen Atemmuster-Analysator (21) und einen Kontur-Analysator (20) und eine Transformations-Funktion (22) und einen Druckprofil-Applikator (23). Insbesondere kann die Steuereinheit alle erfindungsgemäßen Funktionen auch selbststätig ausführen, ohne das eigenständige Funktionseinheiten notwendig sind.

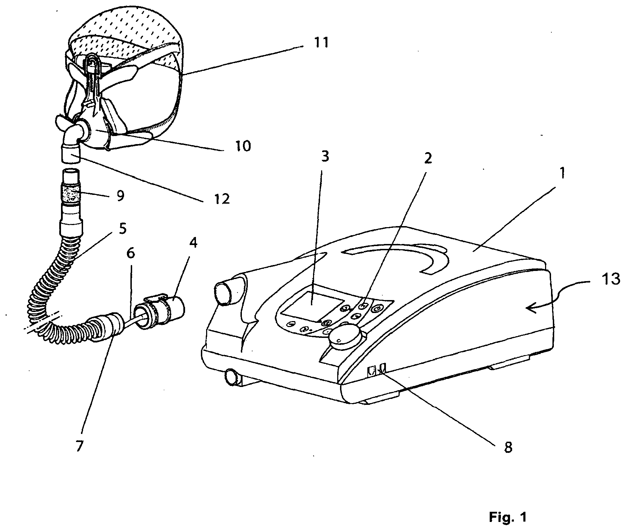

Fig. 1 zeigt den grundsätzlichen Aufbau einer erfindungsgemäßen Vorrichtung zur Beatmung. Im Bereich eines Gerätegehäuses (1) des Beatmungsgerätes (13), mit einer Atemgasquelle im Geräteinnenraum, sind ein Bedienelement (2) und ein Bedien-und Informationssystem (3) bestehend aus einer Anzeige (3), welche eine, beispielsweise berührungsempfindliche, Eingabeeinheit mit zumindest einem Bedienfeld aufweisen kann. Über eine Kopplung (4) wird ein Verbindungsschlauch (5) angeschlossen. Entlang des Verbindungsschlauches (5) kann ein zusätzlicher Druckmessschlauch (6) verlaufen, der über einen Druckeingangsstutzen (7) mit dem Gerätegehäuse (1) verbindbar ist. Zur Ermöglichung einer Datenübertragung weist das Gerätegehäuse (1) zumindest eine Schnittstelle (8) auf. Ein Anfeuchter kann zudem adaptiert werden. Im Bereich einer dem Gerätegehäuse (1) abgewandten Ausdehnung des Verbindungsschlauches (5) ist ein Ausatmungselement (9) angeordnet. Ebenfalls kann ein Ausatemventil verwendet werden.

Fig. 1 zeigt darüber hinaus ein als Beatmungsmaske (10) ausgebildetes Patienten-Interface, das als Nasalmaske realisiert ist. Eine Fixierung im Bereich eines Kopfes eines Patienten kann über eine Kopfhaube (11) erfolgen. Im Bereich seiner dem Verbindungsschlauch (5) zugewandten Ausdehnung weist das Patienten-Interface (10) ein Kupplungselement (12) auf.

Über die Schnittstelle (8) kann die Eingabe und / oder Ausgabe von Daten, wie beispielsweise Totraumvolumen, erfolgen. Die Schnittstellen können kabelgebunden, als Infrarot-Schnittstelle, als Bluetooth-Schnittstelle oder USB realisiert sein. Bevorzugt ist auch ein Kartenschacht vorgesehen. Die Schnittstelle (8) kann auch als LAN-Schnittstelle oder als sonstige Schnittstelle zur Anbindung an das Internet ausgeführt sein. Im Bereich eines Gerätegehäuses kann ein Sauerstoffzuschaltventil an die Vorrichtung zur Beatmung adaptiert werden. Es ist denkbar, das Atemgas zusätzlich mit Sauerstoff anzureichern, um die Patientenversorgung zu verbessern. Statt einer Schnittstelle (8) kann auch eine Vielzahl von Schnittstellen vorgesehen sein.

Über die Schnittstelle (8) - beispielsweise als Kartenschacht oder USB ausgeführt - können auch therapiefremde Daten in das erfindungsgemäße Beatmungsgerät geladen werden beziehungsweise von diesem ausgeführt werden. So ist daran gedacht beispielsweise Fotos oder Videos mittels Speichermedien über die Schnittstelle (8) im Bereich der Anzeige darzustellen. Der Anwender muss - werden vom Gerät externe Speichermedien erkannt - eine Abfrage im Bedienfeld bestätigen, woraufhin die Daten wahlweise im Bereich des Beatmungsgerätes gespeichert oder ausgeführt werden.

Über die Schnittstelle (8) kann die Eingabe und / oder Ausgabe von telemedizinischen Daten erfolgen. Dazu werden über die Schnittstelle beispielsweise Mobilfunk- oder Nahfeldfunkdaten empfangen / gesendet oder WLAN oder Bluetooth oder Netzwerkdaten.

Das erfindungsgemäße Beatmungsgerät (13) ist so ausgelegt, dass es über einen Schlauch und ein Patienteninterface mit einem Patienten verbunden werden kann, um eine Beatmung bereitzustellen. Es umfasst eine Quelle für Atemgas, die beispielsweise als ein Elektromotor mit Gebläserad ausgebildet ist, und eine Einrichtung zur Ermittlung von Fluss, und optional Druck und/oder Volumen, des Atemgases, sowie eine Steuereinheit (14), die so ausgelegt ist, dass sie für jeden Atemzyklus auf Basis von Messsignalen für die Parameter Fluss, und optional Druck und/oder Volumen, und optional auf der Basis eines vorbestimmten Wertes für den Patienten, einen Atemgasdruck bestimmt und die Quelle für Atemgas derart reguliert, dass der Atemgasdruck erzeugt wird.

Die Steuereinheit (14) ist ferner so ausgelegt, dass sie den aktuellen Fluss, und optional Druck und/oder das Volumen, von Atemgas bestimmt und den aktuellen Wert über das mit der Steuereinheit Bedien- und Informationssystem (3) darstellt. Die Steuereinheit (14) ist außerdem so ausgelegt, dass sie bezogen auf einen oder mehrere Parameter Trendveränderungen ihrer Berechnungen über der Zeit bestimmt, wobei die Trendveränderungen auf der Anzeige angezeigt werden können.

Weiterhin vergleicht die Steuereinheit (14) solche ParameterWerte, die von einem Benutzer vorgegeben wurden, beispielsweise obere und untere Druckgrenzen oder eine maximal tolerierbare Anzahl von Apnoen pro Zeiteinheit, oder eine maximal tolerierbare Leckage, mit den aktuellen Werten und generiert eine Benutzer-Information zu Abweichungen von der Vorgabe. Die Benutzerinformation wird bevorzugt über das Bedien- und Informationssystem (3) graphisch visualisiert.

So werden aus dem gemessenen Atemfluss über eine Abnahme des Atem(zeit)volumens für eine Zeitdauer von mindestens 10 s Apnoen und Hypopnoen erkannt. Zusätzlich wird über Druck- und Flussschwankungen Schnarchen, sowie über die inspiratorische Flusskontur Flattening erkannt. Daraus werden für jede ausreichend lange nächtliche Therapie Indizes berechnet, nämlich: AHI (= Anzahl Apnoen + Hypopnoen pro artefaktfreie Therapiedauer), RDI (= Anzahl aller respiratorischen Ereignisse pro artefaktfreie Therapiedauer), Anteil Atemzüge mit Flattening, Anteil Atemzüge mit Schnarchen. Bevorzugt werden auch Daten ermittelt, die auf das Nutzungsverhalten oder auf die Nutzungsdauer des Gerätes durch den Patienten schließen lassen. Diese Daten werden täglich oder wöchentlich oder monatlich ermittelt und gespeichert. Bedarfsweise werden die Nutzungsdaten, gegebenenfalls zusammen mit einer Gerätekennung, über eine Internetverbindung oder Mobilfunkverbindung abgerufen und versendet.

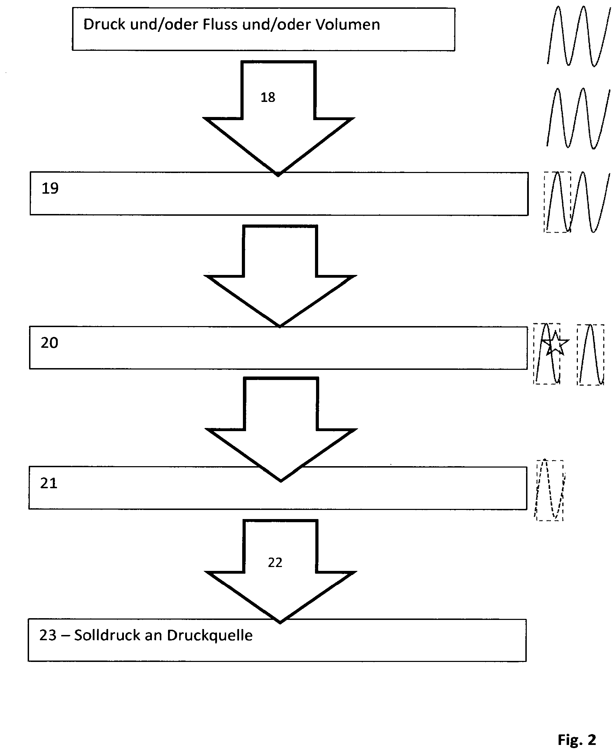

Figur 2 zeigt, wie die erfindungsgemäße Vorrichtung zur Beatmung (13) mit zumindest einer Druckgasquelle für das Atemgas (14) und einer Sensoreinheit (15) zur Bestimmung von Atemgas Fluss, und optional Druck und/oder Volumen, und einer Steuereinheit (16) sowie einem Speicher (17) aus den Fluss-Werten, und optional Druck- und/oder Volumen-Werten, einen Solldruck für die Druckgasquelle bestimmt. Die Steuereinheit (16) weist einen Verlaufs-Analysator (18) auf, der den Verlauf von mindestens einem Signal, das mit dem Fluss, und optional Druck und/oder Volumen, in Zusammenhang steht, kontinuierlich (mindestens mit 1 Hz, bevorzugt > 20 Hz) abtastet, analysiert und im Speicher zwischenspeichert. Die Steuereinheit (16) weist zudem eine Ausschneide-Einheit (19) auf, die definierte oder aus dem Verlauf von mindestens einem Signal, das mit dem Fluss, und optional Druck und/oder Volumen, in Zusammenhang steht, erkannte Zeiträume des Verlaufes mindestens eines Signals aus dem Zwischenspeicher auswählt und an den Atemmuster-Analysator und den Kontur-Analysator übergibt.

Die Steuereinheit (16) weist zudem einen Kontur-Analysator (20) auf, der aus dem Verlauf des aktuellen Zeitraums, der ihm von der Ausschneide-Einheit (19) übergeben wurde, das Vorhandensein oder Nicht-Vorhandensein bestimmter akuter Atmungszustände oder Beatmungszustände erkennt.

Die Steuereinheit (16) weist zudem einen Atemmuster-Analysator (21) auf, der das typische individuelle Atemmuster eines Patienten aus mindestens zwei Verläufen ermittelt, die ihm von der Ausschneide-Einheit übergeben wurden.

Die Steuereinheit (16) weist zudem eine Transformations-Funktion (22) auf, die durch mindestens eine vorgegebene mathematische Abbildung (31) das individuelle Atemmuster in ein für den aktuellen oder darauffolgenden Atemzug gültiges Druckprofil (29) überführt.

Die Steuereinheit (16) weist zudem einen Druckprofil-Applikator (23) auf, der anhand der Ergebnisse des Verlaufs-Analysators das vorgesehene Druckprofil auf den augenblicklichen Verlauf des Atemzuges optimiert und daraus den Solldruck (mindestens 1 Hz, bevorzugt > 20 Hz) an die Druckquelle übergibt.

Der Verlaufs-Analysator (18) erkennt beispielsweise als Signaltypen: Gerätefluss, Patientenfluss, Patientenfluss mit Spülfluss, Druck am Geräteausgang, Druck in der Maske, Druck in den Atemwegen, aktuelles Tidalvolumen, aktuelles alveoläres Tidalvolumen sowie Schätzgrößen für eines dieser Signale oder damit in Zusammenhang stehende Signale. Als mögliche Analysen führt der Verlaufs-Analysator (18) aus: Erkennung der Atemphase, Erkennung des aktuellen Momentes innerhalb des Verlaufes eines Atemzuges, Erkennung von steigendem oder fallendem Volumen gegenüber dem individuellen Atemmuster oder den unmittelbar vorangegangenen Atemzügen, Synchronität von Druckverlauf und Patientenatmung.

Die Ausschneide-Einheit (19) definiert Beginn und Ende der Zeiträume durch beispielsweise: feste Zeitdauern, beispielsweisefeste Sekundenanzahl oder 1/Atemfrequenz, oder durch charakteristische Punkte wie Nulldurchgang mindestens eines der analysierten Signale nach Abzug eines Offsets, Inspirationsbeginn, Exspirationsbeginn, inspiratorische Peakfluss, exspiratorischer Peakfluss, Erreichen eines vorgegebenen Druckwertes, maximale oder minimale Steigung eines Signals oder Nulldurchgang der Steigung, maximale oder minimale Krümmung des Signals oder Nulldurchgang, Beginn der Ende eines kompletten Atemzuges, Erreichen eines bestimmten Volumens.

Der Kontur-Analysator (20) erkennt mögliche Atmungszustände oder Beatmungszustände wie Flattening in der Inspiration durch Obstruktion der oberen Atemwege, Flattening oder Instabilitäten in der Exspiration durch Obstruktion der unteren Atemwege, Sekret, COPD, Masken- oder Mundleckage, Verpasste Atemzüge, Artefaktzustände wie Bewegung, Sprechen, Husten, Schlucken, ..., Synchronität bzw. aktueller Zeitversatz von Druckverlauf und Atmungsverlauf, Aktuelle Atemanstrengung, Aktuelles aktives Wehren gegen die Beatmung, Aktuelles Tidalvolumen, alveoläres Tidalvolumen, Minutenvolumen, alveoläres Minutenvolumen (alveoläre Volumina durch Berücksichtigung eines ventilatorischen Totraums, der manuell vorgegeben oder vom Gerät automatisch geschätzt wird), aktueller Volumenbedarf, Resistance und/oder Compliance der Atemwege und Lunge oder damit in Zusammenhang stehende Werte

Aus mindestens 2 einzelnen Atemmusterverläufen wird vom Atemmuster-Analysator (21) das individuelle Atemmuster des Patienten bestimmt durch gewichtete Mittelung von einzelnen Atemmustern, wobei die Gewichtung von den vom Kontur-Analysator erkannten Zuständen (beispielsweise niedriges Gewicht bei Auftreten von Flattening oder Artefakten, ...) abhängt, und optional durch mindestens einen der folgenden Schritte; Normierung von aktuellen Atemmustern in Zeit und Fluss oder Volumen, Mittelung von einzelnen Atemmustern, Least-Squares Anpassung oder artverwandte Anpassung einer aktuellen Schätzung des individuellen Atemmusters an das tatsächliche Atemmuster der aktuellen Zeiteinheit, Gleitender Mittelwert des Verlaufes mehrerer Zeiteinheiten, Beseitigung eines Zeitversatzes von Atemmustern innerhalb von mindestens 2 Zeiteinheiten, Glättung des Atemmusters, beispielsweise zur Beseitigung von Verfälschungen, beispielsweise durch Flattening

Die mathematische Abbildung (31) der Transformations-Funktion (22) kann beinhalten:
- Vorgaben aufgrund des Krankheitsbildes, beispielsweise ein optimiertes Druckprofil (29) bei restriktiven oder obstruktiven Lungen; das Krankheitsbild kann dabei entweder vom Anwender vorgegeben, oder vom Beatmungsgerät automatisch erkannt werden
- Vorgaben zum Therapieziel, beispielsweise Minimierung der Atemarbeit, Stabilisierung des CO2-Wertes, Optimierung des Patientenkomforts, Vermeidung eines intrinsischen PEEPs bei der Beatmung, Einhaltung eines Zielwertes für Tidalvolumen, alveoläres Tidalvolumen, Minutenvolumen, alveoläres Minutenvolumen, Therapie von Obstruktionen der oberen Atemwege, Stabilisierung des SpO2-Wertes, Sicherstellung eines minimalen oder maximalen Druckhubes, Vermeidung von gefährdenden Situationen, ...; die Vorgaben können dabei vom Anwender gewählt oder fest im Gerät hinterlegt sein
- Fest im Gerät hinterlegte Universalfunktionen zur Überführung eines Atemmusters in ein passendes Druckprofil, beispielsweise aus einer parallelen Aufzeichnung von Druck und Fluss auf einer Zeitachse, aus der korrespondierende Druck- und Flusswerte ablesbar sind.
- Maßnahmen zur Optimierung der Synchronität von Druckprofil und Atemanstrengung des Patienten, beispielsweisedurch Strecken oder Stauchen des Druckprofils
- Maßnahmen zur Beschleunigung der Atmung oder Verlangsamung der Atmung
- Dabei kann die Transformations-Funktion optional auf weitere Signale von außen zugreifen, insb.: SpO2, CO2, Pulswelle, Effort, Herzfrequenz, Eingaben von Beatmungsparametern durch Patient oder medizinisches Personal, EMG der Atemmuskeln, Atemgeräusche, Atemanstrengung
- Oder auf die Ergebnisse des Kontur-Analysators, um beispielsweisedurch Anhebung / Absenkung des Druckprofils auf Obstruktionen der oberen Atemwege zu reagieren, eines der genannten Volumina zu kontrollieren oder durch Beschleunigen / Verlangsamen des Druckprofils die Synchronität von Beatmung und Atemanstrengung des Patienten zu verbessern oder intrinsischen PEEP zu verringern

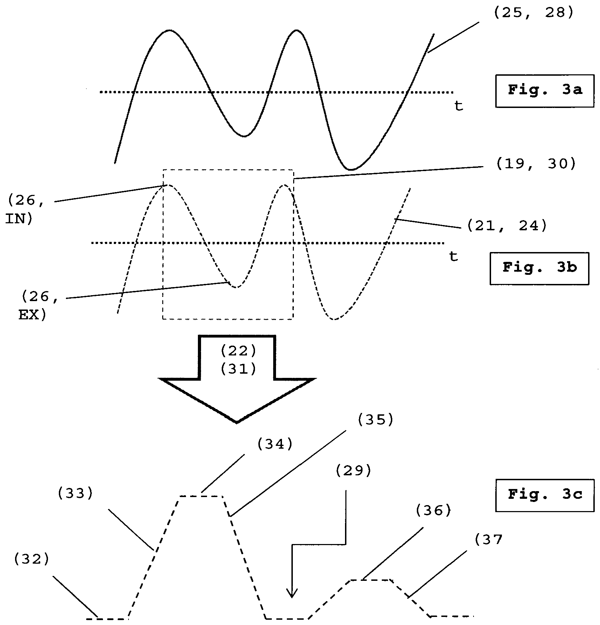

Der Druckprofil-Applikator (23) optimiert alternativ zusätzlich das Druckprofil für den aktuellen Atemverlauf anhand der Ergebnisse des Verlaufs-Analysators:
- Insbesondere um auf Abweichungen des aktuellen Atmungsverlaufes vom individuellen Atemmuster einzugehen
- Die Optimierung kann beispielsweise derart erfolgen, dass die Abweichung unterstützt oder ihr entgegenwirkt
- Abweichungen werden insbesondere bezüglich Höhe von Fluss/Volumen, alveolärem Fluss/Volumen oder Beschleunigung / Verlangsamung der Atmung bestimmt
- Die Optimierung erfolgt beispielsweise durch eine mathematische Abbildung, insbesondere anhand der Ergebnisse des Kontur-Analysators
- Oder durch Skalierung des durch die mathematische Abbildung ermittelten Druckprofils in Höhe oder Dauer / Geschwindigkeit. Figur 3a) zeigt, wie ein vollständiger oder teilweiser Atemzug, beispielsweise zumindest eine Inspiration und eine Exspiration, von der Sensoreinheit (15) zur Bestimmung von Atemgas-Fluss, und optional -Druck und/oder -Volumen, als Signal (25), aufgezeichnet wird. Die Aufzeichnung erfolgt mit beispielsweise > 20 Hz, bevorzugt > 50 Hz oder > 90 Hz oder 100 Hz und wird im Speicher (17) abgelegt. Als Signal für den Beginn der Aufzeichnung kann beispielsweise dienen, der aus dem Atemgasfluss und optional
- Druck erkannte exspiratorischen oder inspiratorische Trigger (Ausatembemühung des Patienten) oder einige Millisekunden nach dem exspiratorischen Trigger.

Alternativ auch ein Nulldurchgang nach dem exspiratorischen Trigger.

Das als Signal (25) wird unter Berücksichtigung der Zeit (t) als Verlauf (28) aufgezeichnet. Der Verlauf (28) kann anschließend mit geringerer Auflösung von beispielsweise unter 90 Hz, bevorzugt unter 50 Hz, besonders bevorzugt unter 40 Hz oder unter 30 Hz verwendet werden.

Der Verlaufs-Analysator (18), tastet ab und/oder analysiert den Verlauf (28) von mindestens einem Signal (25), das mit dem Atemgas-Fluss, und optional -Druck und/oder -Volumen, in Zusammenhang steht oder Atemgas-Fluss, und optional -Druck und/oder -Volumen, ist, und speichert (17) dieses. Der Verlauf (28) des Signals (25) kann dafür aus dem Speicher geladen werden oder der Verlauf des Signals wird "online" während der Bestimmung durch die Sensoreinheit durch den Verlaufs-Analysator (18) bearbeitet und erst dann gespeichert. Der Verlaufs-Analysator (18) ist beispielsweise ausgebildet und eingerichtet, eine Abflachung des Fluss- oder Volumensignals in der Inspiration als Flattening zu erkennen. Der Verlaufs-Analysator (18) ist beispielsweise auch ausgebildet und eingerichtet, aus dem Signal das Volumen, das I zu E Verhältnis, Artefakte, Mundexspiration, COPD-Atmung oder Cheyne-Stokes Atmung zu erkennen.

Figur 3b) zeigt: Die Ausschneide-Einheit (19), wählt definierte, oder aus dem Verlauf (28) erkannte, Zeiträume (30) des Verlaufes mindestens eines Signals (25) aus dem Speicher (17) aus. Die Ausschneide-Einheit (19) kann beispielsweise eine Inspiration, eine Exspiration, einen Patiententrigger, das Volumen, das I zu E Verhältnis, Artefakte, Mundexspiration, COPD-Atmung oder Cheyne-Stokes Atmung auswählen. Der oder die von der Ausschneide-Einheit (19) gewählten oder erkannten Zeiträume (30) werden von dem Atemmuster-Analysator (21) und/oder den Kontur-Analysator (20) verwendet. Dieses kann gleichzeitig oder nacheinander erfolgen.

Der Kontur-Analysator (20) ist eingerichtet und ausgebildet aus dem Verlauf (28) des aktuellen Zeitraums das Vorhandensein oder Nicht-Vorhandensein bestimmter Atmungszustände (26) oder Beatmungszustände (27) zu erkennen. Beispielsweise sind dies eine Inspiration (IN), eine Exspiration (EX), einen Patiententrigger, das Volumen, das I zu E Verhältnis, Artefakte, Mundexspiration, COPD-Atmung oder Cheyne-Stokes Atmung. Beispielsweise sind dies auch die Form der Inspiration und/oder der Exspiration.

Der Atemmuster-Analysator (21) ist eingerichtet und ausgebildet das individuelle Atemmuster (24) eines Patienten aus mindestens zwei Verläufen (28) zu ermitteln. Dabei werden beispielsweise die Form der Inspiration und/oder der Exspiration oder des Atemzuges im Fluss- und optional Volumen- und/oder Druckverlauf von mindestens zwei Verläufen (28) verglichen und zu einer, für den Patienten individuellen, Normkontur gefügt, die das individuelle Atemmuster (24) eines Patienten repräsentiert.

Figur 3c) zeigt: Die Transformations-Funktion (22) überführt das individuelle Atemmuster (24) oder den Verlauf (28) durch mindestens eine mathematische Abbildung (31) in ein Druckprofil (29) für den Patienten. Das Druckprofil (29) berücksichtigt im Wesentlichen beispielsweise die Zeit (t) aus dem Verlauf (28) und/oder die zeitlichen Lage von Atmungszuständen (26) oder Beatmungszuständen (27) in dem Atemmuster (24) / dem Verlauf (28). Das Druckprofil (29) gibt unter Berücksichtigung von Atemmuster (24) oder Verlauf (28) Druckniveaus (32, ...37) oder Druckformen vor, die, im Wesentlichen zeitsynchron zu dem Atemmuster (24) oder dem Verlauf (28), dem Patientenbedarf angepasst sind.

Das Druckprofil (29) definiert beispielsweise einen EPAP (32) der mit einer optionalen, definierten Druckrampe (33) auf den IPAP (34) erhöht wird. Der IPAP wird mit einer optionalen, definierten Druckrampe (35) auf den EPAP (32) erniedrigt. Optional oder ergänzend wird der EPAP (32) mit einer optionalen, definierten Druckrampe auf ein leicht erhöhtes exspiratorisches Niveau (36) angehoben. Das leicht erhöhte exspiratorische Niveau (36) bietet dem Patienten in der Ausatmung einen leichten Gegendruck und dient beispielsweise dazu, Alveolen offen zu halten. Das leicht erhöhte exspiratorische Niveau (36) kann in Abhängigkeit vom Patientenfluss definiert sein. Nach dem Niveau (36) kann über eine optionale, definierte Druckrampe (37) zunächst noch einmal der EPAP (32) vorgegeben werden. Alternativ kann von dem Niveau (36) ausgehend der IPAP (34) - optional über eine definierte Druckrampe (33) - angesteuert werden. Das Druckprofil kann insbesondere für die Niveaus (32) und/oder (34) und/oder (36) Druckwellenformen statt der Druckplateaus vorgeben. Bei dem Profil (29) können auch keine üblichen, abgesetzten Druckniveaus (32, ...37) erkennbar sein. Das Profil (29) verläuft entsprechend dem Atemmuster (24) oder dem Verlauf (28) und ist dem Patientenbedarf optimal angepasst.

Der Druckprofil-Applikator (23) steuert die Druckgasquelle (14), unter Berücksichtigung des Druckprofils (29), an. Der Druckprofil-Applikator (23), anhand der Ergebnisse des Verlaufs-Analysators, optimiert das vorgesehene Druckprofil auf den augenblicklichen Verlauf des Atemzuges und übergibt daraus den augenblicklichen Solldruck kontinuierlich an die Druckquelle.

Der Druckprofil-Applikator (23) steuert die Druckgasquelle (14), unter Berücksichtigung des Druckprofils (29), beispielsweise derart an, dass eine optimierte Synchronisierung mit der Eigenatmung des Patienten erfolgt und/oder eine möglichst komfortable Beatmung vorgegeben wird und/oder die Atemarbeit des Patienten möglichst gering ist und/oder die Bildung eines intrinsischen PEEP verhindert wird und/oder eine bestimmte Minutenventilation erreicht wird und/oder eine antizyklische Servoventilation zur Therapie bestimmter krankhafter Atemmuster erfolgt und/oder Obstruktionen der Atemwege verhindert werden und/oder ein bestimmtes Tidalvolumen erreicht wird.

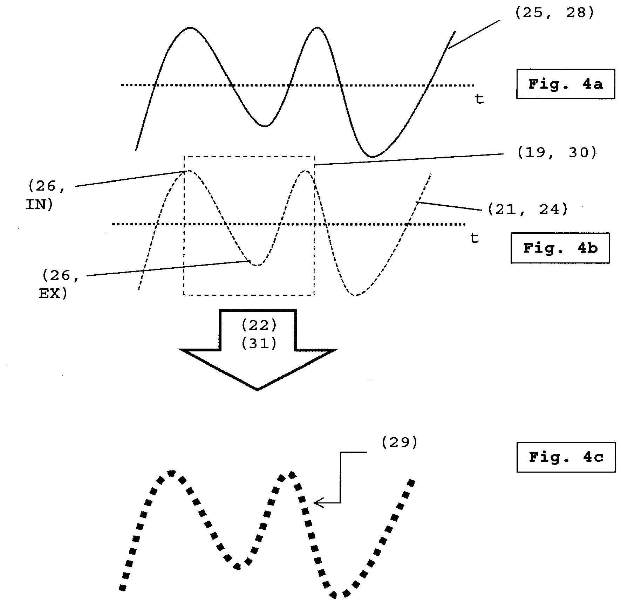

Figur 4 a und b zeigt die beschriebenen Schritte wie in Figur 3 a und b. Figur 4c) zeigt jedoch: Die Transformations-Funktion (22) überführt das individuelle Atemmuster (24) oder den Verlauf (28) durch mindestens eine mathematische Abbildung (31) in ein Druckprofil (29) für den Patienten. Das Druckprofil (29) berücksichtigt im Wesentlichen beispielsweise die Zeit (t) aus dem Verlauf (28) und/oder die zeitlichen Lage von Atmungszuständen (26) oder Beatmungszuständen (27) in dem Atemmuster (24) / dem Verlauf (28). Das Druckprofil (29) gibt unter Berücksichtigung von Atemmuster (24) oder Verlauf (28) und optional Vorgaben eine Druckform oder ein Profil (29) vor. Es sind keine üblichen, abgesetzten Druckniveaus (32, ...37) erkennbar. Das Profil (29) verläuft beispielsweise zeitsynchron zu dem Atemmuster (24) oder dem Verlauf (28) und ist dem Patientenbedarf optimal angepasst.

## Patentansprüche

1. Vorrichtung zur Beatmung (13) mit zumindest einer Druckgasquelle für Atemgas (14) und einer Sensoreinheit (15) zur Bestimmung von Atemgas-Fluss (25) und einer Steuereinheit (16) sowie einem Speicher (17), wobei die Steuereinheit (16) aufweist:
- einen Verlaufs-Analysator (18), der eingerichtet ist, einen Verlauf (28) von mindestens einem durch die Sensoreinheit (15) aufgezeichnetem Signal (25) zu ermitteln und im Speicher (17) zwischenzuspeichern;
- einen Kontur-Analysator (20), der eingerichtet und ausgebildet ist, aus dem Verlauf (28) des aktuellen Zeitraums das Vorhandensein oder Nicht-Vorhandensein bestimmter Atmungszustände (26) oder Beatmungszustände (27) zu erkennen;
- einen Atemmuster-Analysator (21), der eingerichtet und ausgebildet ist ein individuelles Atemmuster (24) eines Patienten aus mindestens zwei der Verläufe (28) zu ermitteln durch eine gewichtete Mittelung von einzelnen Atemmustern, wobei die Gewichtung abhängt von den vom Kontur-Analysator erkannten Zuständen;
- eine Transformations-Funktion (22), die eingerichtet ist, durch mindestens eine mathematische Abbildung (31) das individuelle Atemmuster (24) in ein Druckprofil (29) zu überführen, und dabei auch Vorgaben eines Anwenders, welche über ein Bedienelement (2) oder ein Bedien- und Informationssystem (3) oder eine Schnittstelle (8) der Vorrichtung eingespeichert (17) sind, zu berücksichtigen, wobei
- das Druckprofil (29) das individuelle Atemmuster (24) und die Vorgaben eines Anwenders berücksichtigt und wobei
- das Druckprofil (29) eine Abfolge von Druck-Niveaus und/oder Druckwellenformen und/oder Druckplateaus und/oder Druckrampen aufweist, die im Wesentlichen, zumindest abschnittsweise, korrespondierende Abschnitte, wie beispielsweise die Frequenz und/oder das I/E-Verhältnis und/oder die das Minutenvolumen und/oder Inspirationen und Exspirationen, der Patientenatmung aus dem Verlauf (28) und/oder dem Atemmuster (24) wiederspiegeln; und
- einen Druckprofil-Applikator (23), der eingerichtet ist, die Druckgasquelle (14) unter Berücksichtigung des Druckprofils (29) anzusteuern, wobei
- der Druckprofil-Applikator (23), anhand der Ergebnisse des Verlaufs-Analysators (18) das vorgesehene Druckprofil (29) auf den augenblicklichen Verlauf (28) des Atemzuges optimiert und daraus den augenblicklichen Solldruck kontinuierlich an die Druckgasquelle (14) übergibt.

2. Vorrichtung nach Anspruch 1, **dadurch gekennzeichnet, dass** die Sensoreinheit (15) eingerichtet ist, Atemgas-Fluss (25) mit einer Auflösung von mindestens 40 Hz oder größer als 60 Hz aufzuzeichnen.

3. Vorrichtung nach zumindest einem der vorhergehenden Ansprüche, **dadurch gekennzeichnet, dass** die Steuereinheit eingerichtet ist, den Verlauf (28) von dem mindestens einen Signal (25) mit reduzierter Auflösung von unter 40 Hz oder unter 30 Hz zwischenzuspeichern (17).

4. Vorrichtung nach zumindest einem der vorhergehenden Ansprüche, **dadurch gekennzeichnet, dass** die Vorrichtung eine Ausschneide-Einheit (19) umfasst, wobei die Ausschneide-Einheit (19) eingerichtet und ausgebildet ist, definierte oder aus dem Verlauf (28) erkannte Zeiträume (30), aus dem Speicher (17), an den Atemmuster-Analysator (21) und/oder den Kontur-Analysator (20) zu übergeben.

5. Vorrichtung nach zumindest einem der vorhergehenden Ansprüche, **dadurch gekennzeichnet, dass** der Kontur-Analysator (20) eingerichtet ist, als Atmungszustände (26) oder Beatmungszustände (27) zu erkennen: eine Inspiration und/oder eine Exspiration und/oder einen Patiententrigger und/oder das Volumen und/oder das Inspiration zu Exspiration (I zu E) Verhältnis und/oder Artefakte und/oder Mundexspiration und/oder COPD (chronic obstructive pulmonary disease)-Atmung und/oder Cheyne-Stokes Atmung.

6. Vorrichtung nach zumindest einem der vorhergehenden Ansprüche, **dadurch gekennzeichnet, dass** der Kontur-Analysator (20) eingerichtet ist, bestimmte Atmungszustände (26) oder Beatmungszustände (27) durch Mustervergleich zu erkennen.

7. Vorrichtung nach zumindest einem der vorhergehenden Ansprüche, **dadurch gekennzeichnet, dass** die Transformations-Funktion (22) eingerichtet ist, einen aktuellen SignalVerlauf (28) und das gespeicherte individuelle Atemmuster (24) zu verwenden.

8. Vorrichtung nach zumindest einem der vorhergehenden Ansprüche, **dadurch gekennzeichnet, dass** der Druckprofil-Applikator (23) eingerichtet ist, die Druckgasquelle (14), unter Berücksichtigung des Druckprofils (29), derart anzusteuern, dass eine optimierte Synchronisierung mit der Eigenatmung des Patienten erfolgt und/oder eine möglichst komfortable Beatmung vorgegeben wird und/oder die Atemarbeit des Patienten möglichst gering ist und/oder die Bildung eines intrinsischen PEEP verhindert wird und/oder eine bestimmte Minutenventilation erreicht wird und/oder eine antizyklische Servoventilation zur Therapie bestimmter krankhafter Atemmuster erfolgt und/oder Obstruktionen der Atemwege verhindert werden und/oder ein bestimmtes Tidalvolumen erreicht wird.

9. Vorrichtung zur Beatmung (13) nach Anspruch 4 oder einem der Ansprüche 5 bis 8 insofern als abhängig vom Anspruch 4, **dadurch gekennzeichnet, dass**
- der Verlaufs-Analysator (18) eingerichtet ist, den Verlauf (28) von dem mindestens einen Signal (25) abzutasten und zu analysieren, und
- die Ausschneide-Einheit (19) eingerichtet ist, die definierten oder aus dem Verlauf (28) erkannten Zeiträume (30) auszuwählen, wobei die Zeiträume Zeiträume des Verlaufes des mindestens einen Signals (25) sind.

10. Vorrichtung zur Beatmung (13) nach Anspruch 4 oder einem der Ansprüche 5 bis 8 insofern als abhängig vom Anspruch 4, **dadurch gekennzeichnet, dass**
- der Verlaufs-Analysator (18) eingerichtet ist, den Verlauf von dem mindestens einen Signal abzutasten und zu analysieren,
- die Ausschneide-Einheit (19) eingerichtet ist, die definierten oder aus dem Verlauf erkannten Zeiträume aus dem Zwischenspeicher auszuwählen und an den Atemmuster-Analysator und an den Kontur-Analysator zu übergeben, wobei die Zeiträume Zeiträume des Verlaufes des mindestens einen Signals sind,
- der Kontur-Analysator (20) eingerichtet ist, aus dem Verlauf des aktuellen Zeitraums, der ihm von der Ausschneide-Einheit übergeben wurde, das Vorhandensein oder Nicht-Vorhandensein bestimmter akuter Atmungszustände oder Beatmungszustände zu erkennen,
- der Atemmuster-Analysator (21) eingerichtet ist, das typische individuelle Atemmuster eines Patienten aus mindestens zwei Verläufen zu ermitteln, die ihm von der Ausschneide-Einheit übergeben wurden, und
- die Transformations-Funktion (22) eingerichtet ist, durch die mindestens eine mathematische Abbildung das individuelle Atemmuster in ein für den aktuellen oder darauffolgenden Atemzug gültiges Druckprofil zu überführen.

## Claims

1. A ventilation device (13) having at least one compressed gas source for respiratory gas (14) and a sensor unit (15) for determining respiratory gas flow (25) and a control unit (16) as well as a memory (17), wherein the control unit (16) comprises:
- a course analyzer (18) which is configured to ascertain a course (28) of at least one signal (25) recorded by the sensor unit (15) and to temporarily store said course in the memory (17);
- a contour analyzer (20) which is configured and designed to recognize the presence or absence of particular respiratory states (26) or ventilation states (27) from the course (28) of the current time period;
- a breathing pattern analyzer (21) which is configured and designed to ascertain an individual breathing pattern (24) of a patient from at least two of the courses (28) by means of weighted averaging of individual breathing patterns, wherein the weighting depends on the states recognized by the contour analyzer;
- a transformation function (22) which is configured to convert the individual breathing pattern (24) into a pressure profile (29) by means of at least one mathematical map (31), and in the process also taking into account specifications of a user which are read in (17) via an operating element (2) or an operating and information system (3) or an interface (8) of the device, wherein
- the pressure profile (29) takes into account the individual breathing pattern (24) and the specifications of a user and wherein
- the pressure profile (29) comprises a sequence of pressure levels and/or pressure waveforms and/or pressure plateaus and/or pressure ramps which substantially reflect, at least partially, corresponding parts, for example the frequency and/or the I/E ratio and/or the minute volume and/or inspirations and expirations, of the patient's breathing from the course (28) and/or the breathing pattern (24); and
- a pressure profile applicator (23) which is configured to actuate the compressed gas source (14) taking into account the pressure profile (29), wherein
- the pressure profile applicator (23) optimizes the provided pressure profile (29) for the momentary course (28) of the breath based on the results of the course analyzer (18) and from that continuously transfers the momentary target pressure to the compressed gas source (14).

2. The device according to claim 1, **characterized in that** the sensor unit (15) is configured to record the respiratory gas flow (25) with a resolution of at least 40 Hz or greater than 60 Hz.

3. The device according to at least one of the preceding claims, **characterized in that** the control unit is configured to temporarily store (17) the course (28) of the at least one signal (25) with a reduced resolution of less than 40 Hz or less than 30 Hz.

4. The device according to at least one of the preceding claims, **characterized in that** the device comprises a cutting unit (19), wherein the cutting unit (19) is configured and designed to transfer defined time periods (30) or those recognized from the course (28), from the memory (17) to the breathing pattern analyzer (21) and/or the contour analyzer (20).

5. The device according to at least one of the preceding claims, **characterized in that** the contour analyzer (20) is configured to recognize the following as respiratory states (26) or ventilation states (27): an inspiration and/or an expiration and/or a patient trigger and/or the volume and/or the inspiration to expiration (I to E) ratio and/or artifacts and/or mouth expiration and/or COPD (chronic obstructive pulmonary disease) breathing and/or Cheyne-Stokes breathing.

6. The device according to at least one of the preceding claims, **characterized in that** the contour analyzer (20) is configured to recognize particular respiratory states (26) or ventilation states (27) by means of pattern comparison.

7. The device according to at least one of the preceding claims, **characterized in that** the transformation function (22) is configured to use a current signal course (28) and the stored individual breathing pattern (24).

8. The device according to at least one of the preceding claims, **characterized in that** pressure profile applicator (23) is configured to actuate the compressed gas source (14), taking into account the pressure profile (29), such that an optimized synchronization with the patient's own breathing takes place and/or ventilation that is as comfortable as possible is specified and/or the breathing work of the patient is minimized and/or the formation of an intrinsic PEEP is prevented and/or a particular minute ventilation is achieved and/or anti-cyclical servoventilation takes place for treating particular pathological breathing patterns and/or obstructions of the airways are prevented and/or a particular tidal volume is achieved.

9. The ventilation device (13) according to claim 4 or any one of claims 5 to 8 insofar as they are dependent on claim 4,
**characterized in that**
- the course analyzer (18) is configured to sample and analyze the course (28) of the at least one signal (25), and
- the cutting unit (19) is configured to select the defined time periods (30) or those recognized from the course (28), wherein the time periods are time periods of the course of the at least one signal (25).

10. The ventilation device (13) according to claim 4 or any one of claims 5 to 8 insofar as they are dependent on claim 4,
**characterized in that**
- the course analyzer (18) is configured to sample and analyze the course of the at least one signal,
- the cutting unit (19) is configured to select the defined time periods or those recognized from the course, from the temporary memory and to transfer said time periods to the breathing pattern analyzer and to the contour analyzer, wherein the time periods are time periods of the course of the at least one signal,
- the contour analyzer (20) is configured to recognize the presence or absence of particular acute respiratory states or ventilation states from the course of the current time period that was transferred to said contour analyzer from the cutting unit,
- the breathing pattern analyzer (21) is configured to ascertain the typical individual breathing pattern of a patient from at least two courses which were transferred to said breathing pattern analyzer from the cutting unit, and
- the transformation function (22) is configured to convert the individual breathing pattern into a pressure profile that is valid for the current or subsequent breath by means of the at least one mathematical map.

## Revendications

1. Dispositif de ventilation (13) doté d'au moins une source de gaz sous pression pour du gaz respiratoire (14) et d'une unité de capteur (15) destinée à déterminer d'un flux de gaz respiratoire (25) et d'une unité de commande (16) ainsi que d'une mémoire (17), dans lequel l'unité de commande (16) présente :
- un analyseur d'évolution (18), lequel est conçu pour détecter une évolution (28) d'au moins un signal (25) enregistré par l'unité de capteur (15) et pour le stocker temporairement dans la mémoire (17) ;
- un analyseur de contour (20), lequel est conçu et configuré pour reconnaître l'existence ou la non-existence de certains troubles respiratoires (26) ou troubles de ventilation (27) à partir de l'évolution (28) de la période actuelle ;
- un analyseur de modèle respiratoire (21), lequel est conçu et configuré pour détecter un modèle respiratoire individuel (24) d'un patient à partir d'au moins deux des évolutions (28), par une moyenne pondérée de différents modèles respiratoires, dans lequel la pondération dépend de troubles reconnus par l'analyseur de contour ;
- une fonction de transformation (22), laquelle est conçue pour convertir le modèle respiratoire individuel (24) en un profil de pression (29) au moyen d'au moins une représentation mathématique (31), et pour ainsi prendre également en compte des spécifications d'un utilisateur, lesquelles sont mémorisées (17) par le biais d'un élément de contrôle (2) ou d'un système de contrôle et d'information (3) ou d'une interface (8) du dispositif, dans lequel
- le profil de pression (29) tient compte du modèle respiratoire individuel (24) et des spécifications d'un utilisateur et dans lequel
- le profil de pression (29) présente une série de niveaux de pression et/ou de formes d'onde de pression et/ou de plateaux de pression et/ou de rampes de pression, lesquels reflètent essentiellement, du moins partiellement, des parties correspondantes, telles que par exemple la fréquence et/ou le rapport I/E et/ou le volume par minute et/ou les inspirations et expirations, de la respiration du patient à partir de l'évolution (28) et/ou du modèle respiratoire (24) ; et
- un applicateur de profil de pression (23), lequel est conçu pour actionner la source de gaz sous pression (14) en tenant compte du profil de pression (29), dans lequel
- l'applicateur de profil de pression (23), à l'aide des résultats de l'analyseur d'évolution (18), optimise le profil de pression (29) prévu sur l'évolution instantanée (28) du souffle et transmet en continu la pression de consigne instantanée ainsi obtenue à la source de gaz sous pression (14).

2. Dispositif selon la revendication 1, **caractérisé en ce que** l'unité de capteur (15) est conçue pour enregistrer un flux de gaz respiratoire (25) avec une résolution d'au moins 40 Hz ou supérieure à 60 Hz.

3. Dispositif selon l'une au moins des revendications précédentes, **caractérisé en ce que** l'unité de commande est conçue pour stocker temporairement (17) l'évolution (28) d'au moins un signal (25) avec une résolution réduite inférieure à 40 Hz ou inférieure à 30 Hz.

4. Dispositif selon l'une au moins des revendications précédentes, **caractérisé en ce que** le dispositif comporte une unité de découpage (19), dans lequel l'unité de découpage (19) est conçue et configurée pour transmettre des périodes (30) définies ou reconnues à partir de l'évolution (28), depuis la mémoire (17), à l'analyseur de modèle respiratoire (21) et/ou à l'analyseur de contour (20).

5. Dispositif selon l'une au moins des revendications précédentes, **caractérisé en ce que** l'analyseur de contour (20) est conçu pour reconnaître, en tant que troubles respiratoires (26) ou troubles de ventilation (27) : une inspiration et/ou une expiration et/ou un déclencheur du patient et/ou le volume et/ou le rapport de l'inspiration à l'expiration (I à E) et/ou des artefacts et/ou une expiration buccale et/ou une respiration à BPCO (bronchopneumopathie chronique) et/ou une respiration Cheynes-Stokes.

6. Dispositif selon l'une au moins des revendications précédentes, **caractérisé en ce que** l'analyseur de contour (20) est conçu pour reconnaître, par comparaison de modèles, certains troubles respiratoires (26) ou troubles de ventilation (27).

7. Dispositif selon l'une au moins des revendications précédentes, **caractérisé en ce que** la fonction de transformation (22) est conçue pour utiliser une évolution de signal actuelle (28) et le modèle respiratoire individuel (24) stocké.

8. Dispositif selon l'une au moins des revendications précédentes, **caractérisé en ce que** l'applicateur de profil de pression (23) est conçu pour actionner la source de gaz sous pression (14), en tenant compte du profil de pression (29), de manière à obtenir une synchronisation optimisée avec la respiration propre du patient et/ou de manière à définir une ventilation la plus confortable possible et/ou de manière à réduire autant que possible l'effort respiratoire du patient et/ou de manière à empêcher la formation d'une PEP intrinsèque et/ou de manière à atteindre une certaine ventilation par minute et/ou de manière à obtenir une ventilation auto-asservie anticyclique pour le traitement de certains modèles respiratoires pathologiques et/ou de manière à empêcher des obstructions des voies respiratoires et/ou de manière à atteindre un volume courant déterminé.

9. Dispositif de ventilation (13) selon la revendication 4 ou l'une des revendications 5 à 8 dans la mesure où celle-ci dépend de la revendication 4, **caractérisé en ce que**
- l'analyseur d'évolution (18) est conçu pour explorer et analyser l'évolution (28) d'au moins un signal (25), et
- l'unité de découpage (19) est conçue pour sélectionner les périodes (30) définies ou reconnues à partir de l'évolution (28), dans lequel les périodes sont des périodes de l'évolution de l'au moins un signal (25).

10. Dispositif de ventilation (13) selon la revendication 4 ou selon l'une des revendications 5 à 8 dans la mesure où celle-ci dépend de la revendication 4, **caractérisé en ce que**
- l'analyseur d'évolution (18) est conçu pour explorer et analyser l'évolution d'au moins un signal,
- l'unité de découpage (19) est conçue pour sélectionner les périodes définies ou reconnues à partir de l'évolution et pour les transmettre à l'analyseur de modèle respiratoire et à l'analyseur de contour, dans lequel les périodes sont des périodes de l'évolution de l'au moins un signal,
- l'analyseur de contour (20) est conçu pour reconnaître, à partir de l'évolution de la période actuelle transmise par l'unité de découpage, l'existence ou la non-existence de certains troubles respiratoires aigus ou troubles de ventilation,
- l'analyseur de modèle respiratoire (21) est conçu pour détecter le modèle respiratoire individuel typique d'un patient à partir d'au moins deux évolutions transmises par l'unité de découpage, et
- la fonction de transformation (22) est conçue pour convertir le modèle respiratoire individuel en un profil de pression valable pour le souffle actuel ou suivant au moyen de l'au moins une représentation mathématique.
